# EUROPÄISCHE PATENTANMELDUNG

(11) **EP 0 884 380 A2**
(43) Veröffentlichungstag der Anmeldung: **16.12.1998**
(21) Anmeldenummer: 98109729.8
(22) Anmeldetag: 28.05.1998
(51) Int. Cl.: C11D 3/37, A61K 7/06, A61K 7/16, A61K 7/48

(54) **Milde alkylpolyglucosid-freie Tensidzubereitungen enthaltend hydrophob modifizierte Polyasparaginsäurederivate**

(30) Priorität: 11.06.1997 DE 19724590
(71) Anmelder: Th. Goldschmidt AG, 45127 Essen (DE)
(72) Erfinder: Grüning, Burghard, Dr., 45134 Essen (DE); Simpelkamp, Jörg, Dr., 45130 Essen (DE); Weitemeyer, Christian, Dr., 45134 Essen (DE)

(57) **Zusammenfassung**

Diese Erfindung beschreibt tensidische Zubereitungen, enthaltend ein oder mehrere hydrophob modifizierte Polyasparaginsäurederivate, und ein oder mehrere weitere Tenside aus der Gruppe der nichtionischen, kationischen, amphoteren oder zwitterionischen Tenside sowie deren Mischungen oder wenigstens ein anionisches Tensid in Kombination mit wenigstens einem anionischen, kationischen, nichtionischen, zwitterionischen und/oder amphoteren Tensid, neben üblichen Hilfs- und Zusatzstoffen. Die Mischungen sind frei von Alkylpolyglucosiden, Alkenylpolyglucosiden und Fettsäure-N-alkyl-polyhydroxyalkylamiden.

## Beschreibung

Diese Erfindung beschreibt tensidische Zubereitungen, enthaltend ein oder mehrere hydrophob modifizierte Polyasparaginsäurederivate, und ein oder mehrere weitere Tenside aus der Gruppe der nichtionischen, kationischen, amphoteren oder zwitterionischen Tenside sowie deren Mischungen oder wenigstens ein anionisches Tensid in Kombination mit wenigstens einem anionischen, kationischen, nichtionischen, zwitterionischen und/oder amphoteren Tensid, neben üblichen Hilfs- und Zusatzstoffen` Die Mischungen sind frei von Alkylpolyglucosiden, Alkenylpolyglucosiden und Fettsäure-N-alkyl-polyhydroxyalkylamiden.

Die Mildheit und dermatologische Verträglichkeit von tensidischen Zubereitungen spielt in verschiedenen Anwendungsgebieten, insbesondere aber auf dem kosmetischen Sektor, eine zunehmend bedeutsame Rolle. Dabei sind Tenside mit naturnahen Grundstrukturen von besonderem Interesse, wie sie beispielsweise die in den letzten Jahren in den Blickpunkt des Interesses getretenen Alkylpolyglucoside oder Fettsäure-N-alkylglucamide besitzen. So beschreibt etwa DE 43 19 699 A besonders milde Tensidmischungen aus Alkyloligoglucosiden. DE 195 24 097 A1 beschreibt kosmetische Mittel auf Basis von Alkyl- oder Alkenyloligoglucosiden oder Fettsäure-N-alkylpolyhydroxyalkylamiden, und Fettsäure-Eiweiß-Kondensaten oder Polyasparaginsäurederivaten.

Diese Tensidklassen besitzen jedoch, neben einer Reihe von Vorzügen beträchtliche Nachteile, insbesondere die aufgrund der schlechteren Wasserlöslichkeit und höheren Substantivität im Vergleich zu anderen Tensiden geringere Reinigungswirkung sowie besonders die aufgrund des naturnahen Aufbaus vergrößerte Gefahr des mikrobiellen Befalls derartiger Tensidzubereitungen und den dadurch üblicherweise erforderlichen erhöhten Aufwand an Konservierungsmitteln. Diese Probleme sind geringer bei Verwendung anderer Tensidklassen, beispielsweise von anionischen Tensiden, wie beispielsweise Alkylsulfaten oder Alkylethersulfaten, welche allerdings eine erhöhte haut- und schleimhautreizende Wirkung aufweisen. Die dermatologischen Eigenschaften derartiger Zubereitungen lassen sich zwar oft durch den Einsatz von Sekundärtensiden verbessern wie beispielsweise Betainen (beschrieben beispielsweise in DE 33 05 197 A, DE 30 11 549 A), wobei allerdings oft Einschränkungen in den Anwendungseigenschaften in Kauf genommen werden müssen.

Polyasparaginsäure, welche als biologisch abbaubares Komplexierungsmittel und Cobuilder in Waschmitteln in den Blickpunkt des Interessen getreten ist, lässt sich bekanntermaßen durch alkalische Hydrolyse aus der unmittelbaren Synthesevorstufe Polysuccinimid (PSI, Anhydropolyasparaginsäure), erhalten, welches beipielsweise durch thermische Kondensation der Asparaginsäure oder aus Ammoniak und Maleinsäure gewonnen werden kann.

Die von verschiedenen Arbeitsgruppen beschriebene Umsetzung von Polysuccinimid mit Aminen führt zu Polyasparaginsäureamiden (Kovacs et al., J. Med. Chem. 1967, 10, 904-7; Neuse, Angew. Makromol. Chem. 1991, 192, 35-50). Neri et al. führen die Ringöffnung von PSI mit Ethanolamin durch und erhalten Hydroxyethylaspartamide (J. Med. Chem. 1973, 16, 893-897, Macromol. Synth. 1982, 8, 25-29). DE 37 00 128 A und EP 0 458 079 A beschreiben die nachfolgende Veresterung derartiger Hydroxyethylderivate mit Carbonsäurederivaten und die Verwendung der Produkte in Ultraschallkontrastmitteln und in Wirkstoffdepotzubereitungen.

Es hat sich die Aufgabe gestellt, milde und reizarme Zubereitungen ohne Alkylpolyglucoside oder Fettsäure-N-alkylglucamide zu finden, die sich leicht konservieren lassen bzw. in nicht konservierten Systemen das Keimwachstum behindern. Diese Aufgabe wird gelöst durch tensidische Zubereitungen für Reinigungsmittel und/oder kosmetische Mittel, enthaltend ein oder mehrere hydrophob modifizierte Polyasparaginsäurederivate und ein oder mehrere weitere Tenside aus der Gruppe der nichtionischen, kationischen, amphoteren oder zwitterionischen Tenside und gegebenenfalls ein oder mehrere weitere Tenside aus der Gruppe der anionischen Tenside, sowie deren Mischungen, ausgenommen zuckerbasierende Tenside aus den Klassen der Alkyl- oder Alkenylpolyglucoside oder Fettsäure-N-alkyl-polyhydroxyalkylamide, neben üblichen Hilfs- und/oder Zusatzstoffen.

Alternativ wird die Aufgabe gelöst durch tensidische Zübereitungen für Reinigungsmittel und/oder kosmetische Mittel, enthaltend ein oder mehrere hydrophob modifizierte Polyasparaginsäurederivate und wenigstens zwei weitere Tenside aus der Gruppe der anionischen Tenside sowie deren Mischungen, neben üblichen Hilfs- und/oder Zusatzstoffen.

Alle gegebenen Angaben zur Zusammensetzung der polymeren Produkte beziehen sich vorzugsweise wie üblich auf die mittlere Zusammensetzung der Polymerketten.

Erfindungsgemäß eingesetzte Polyasparaginsäurederivate sind beispielsweise copolymere Polyasparaginsäureester, welche durch eine Polyasparaginsäurestruktur gekennzeichnet sind, bei der die Seitenketten partiell als freie Carbonsäure- bzw. Carboxylatgruppen vorliegen und partiell mit einem oder mehreren Alkoholen mit 1-24 C-Atomen, darunter vorzugsweise mindestens ein langkettiger Alkohol, insbesondere mit 6-18 C-Atomen, oder dessen Derivaten verestert sind.

Ein Syntheseweg der copolymeren Polyasparaginsäureester verläuft über die Ringöffnung von Polysuccinimid mit kurzkettigen Alkoholen, die nachträgliche Umesterung der Produkte mit lankettigen Alkoholen, insbesondere Fettalkoholen und abschliessende Hydrolyse zu den gewünschten copolymeren Polyasparaginsäureestern wie in der DE 195 45 678 A (Püblikationsdatum: 12. Juni 1997) beschrieben, welche hier expressis verbis einbezogen wird.

Ein weiterer Syntheseweg zu den erfindungsgemäß eingesetzten Polyasparaginsäureestern ist die Veresterung von Polyasparaginsäure und ihrer aktivierten Carbonylanaloga, wie Carbonsäureester, -amide, -halogenide und Anhydride mit Alkoholen mit 1 bis 5 C-Atomen nach den üblichen Veresterungsmethoden, gefolgt von der Umesterung mit Alkoholen mit 2 bis 30 C-Atomen, vorzugsweise 6 bis 24 C-Atomen, vorzugsweise in Gegenwart von dem Stand der Technik entsprechenden Katalysatoren, beispielsweise organischen und/oder anorganische Säuren, Lewis-Säuren wie beispielsweise Titan- oder Zinnverbindungen, Basen, oder saure oder basische mineralische Festphasenkatalysatoren. Ein weiterer Syntheseweg ist die direkte Veresterung von Polyasparaginsäure oder den gemäß dem Stand der Technik bei aktivierten Carbonsäuren aktivierten Polyasparaginsäurederivaten wie beispielsweise den Säurechloriden mit Alkoholen mit 6 bis 24 C-Atomen, oder deren üblichen Derivaten, nach den dem Fachmann bekannten Veresterungsmethoden, gegebenenfalls in Gegenwart geeigneter Katalysatoren, Lösungsmittel und/oder löslichkeitsfördernder Agenzien. Die Veresterungsreaktionen können gegebenenfalls in Gegenwart von den Stand der Technik entsprechenden Kupplungsreagenzien wie beispielsweise Carbodiimiden durchgeführt werden. Die beschriebenen Ester können zur Freisetzung der Carboxyl- bzw. Carboxylatgruppen einem nachfolgenden Hydrolyseschritt, beispielsweise mit Alkalimetallhydroxiden, unterworfen werden.

Der Veresterungsgrad der erfindungsgemäß eingesetzten partiellen Polyasparaginsäureester kann vorzugsweise im Mittel 1 bis 95 Mol.-%, insbesondere 5 bis 75 Mol.-%, besonders vorzugsweise 10 bis 50 Mol.-%, bezogen auf die monomeren Einheiten, betragen.

Die genannten Herstellungsverfahren sind sowohl für reine Polyasparaginsäure als auch für polyasparaginsäurereiche aminosäurepolymere, beispielsweise Copolymere aus Asparaginsäure und weitere Aminosäuren aus der Gruppe der proteinogenen Aminosäuren wie beispielsweise Glutaminsäure, und/oder der nicht-proteinogenen Aminsäuren wie beispielsweise m-Amino-1-carbonsäuren anwendbar.

Die Reaktion kann optional in Gegenwart von verträglichkeitsfördernden Agenzien durchgeführt werden. Dieses können grenzflächenaktive Verbindungen sein, beispielsweise Anlagerungsprodukte von 1 bis 30 Mol Ethylenoxid und /oder 0 bis 5 mol Propylenoxid an C₁₂-C₃₀-Fettalkohole und Wollwachsalkohole; Ethylenoxidanlagerungsprodukte von Glycerinmono- und -diestern und Sorbitanmono- und diestern von gesättigten und ungesättigten Fettsäuren mit 6 bis 22 C-Atomen; Anlagerungsprodukte von 2 bis 30 Mol Ethylenoxid und /oder 0 bis 5 mol Propylenoxid an Fettsäuren mit 12 bis 22 C-Atomen und an Alkylphenole mit 8 bis 15 C-Atomen in der Alkylgruppe; C₁₂-C₁₈-Fettsäurepartialester von Anlagerungsprodukten von 1 bis 30 Mol Ethylenoxid an Glycerin; Anlagerungsprodukte von Ethylenoxid an Fette und Öle, beispielsweise Rizinusöl oder gehärtetes Rizinusöl; Partialester von gesättigten oder ungesättigten C₁₂-C₂₂-Fettsäuren, auch verzweigte oder hydroxysubstituierte, mit Polyolen, beispielsweise Ester von Glycerin, Ethylenglykol, Polyalkylenglykolen, Pentaerythrit, Polyglycerin, Zuckeralkoholen wie Sorbit und Polyglucosiden wie Cellulose; Polysiloxan-Polyalkyl-Polyether-Copolymere und deren Derivate sowie hydrophob modifizierte Polyasparaginsäurederivate, beispielsweise teilveresterte Polyasparaginsäuren, teilveresterte Polyasparaginsäure-co-Glutaminsäuren oder Kondensate aus Maleinsäuremonoestern und Ammoniak, beispielsweise hergestellt nach dem erfindungsgemäßen Verfahren oder nach DE 195 45 678 A, wobei das Herstellungsverfahren der genannten Polyaminosäurederivate keinen Einfluß auf deren verträglichkeitsvermittelnde Wirkung hat. Gegebenenfalls kann auch ein gewisser Teil der Produktmischung im Reaktor verbleiben und als Lösungsvermittler für eine folgende Umsetzung dienen.

Als verträglichkeits- bzw. löslichkeitsvermittelnde Agenzien können auch kationische Tenside, beispielsweise aus der Gruppe der quartären Ammoniumverbindungen, quaternisierten Proteinhydrolysate, Alkylamidoamine, quartären Esterderivate, quartären Silikone, quartären Zucker- und Polysaccharidderivate, und Mischungen daraus, anionische Tenside beispielsweise aus der Gruppe der Sulfate, Sulfonate, Carboxylate sowie Mischungen derselben, beispielsweise Alkylbenzolsulfonate, α-Olefinsulfonate, α-sulfonierte Fettsäureester, Fettsäureglycerinestersulfate, Paraffinsulfonate, Alkylsulfate, Alkylpolyethersulfate, Sulfobernsteinsäurealkylester, Fettsäuresalze (Seifen), Fettsäureester der Polymilchsäure, N-Acylaminosäureester, N-Acyltaurate, Acylisethionate, Ethercarboxylate, Monoalkylphosphate, N-Acylaminosäurederivate wie N-Acylaspartate oder N-Acylglutamate, N-Acylsarcosinate,amphotere oder zwitterionische Tenside wie beispielsweise Alkylbetaine, Alkylamidoalkylbetaine des Typs Cocoamidopropylbetain, Sulfobetaine, Phosphobetaine, Sultaine und Amidosultaine, Imidazoliniumderivate, Amphoglycinate, oder nichtionische Tenside wie beispielsweise oxethylierte Fettalkohole, oxethylierte Alkylphenole, oxethylierte Fettsäureester, oxethylierte Mono-, Di- oder Triglyceride oder Polyalkylenglykolfettsäureester, Zuckerester, z.B. Fettsaureester der Saccharose, Fructose oder des Methylglucosids, Sorbitolfettsäureester und Sorbitanfettsäureester (gegebenenfalls oxethyliert), Polyglycerinester, Fettsäurealkanolamide, langkettige tertiäre Aminoxide oder Phosphinoxide sowie Dialkylsulfoxide enthalten sein, ausgenommen Alkyl- oder Alkenylpolyglucoside sowie Fettsäure-N-alkylpolyhydroxyalkylamide.

Vorzugsweise verbleiben die verträglichkeitsfördernden Agenzien im Produkt.

Weiterhin können erfindungsgemäß copolymere Polyasparaginsäurederivate eingesetzt werden, welche aus Asparaginsäureeinheiten mit freien Carbonsäure- bzw. Carboxylatseitenketten, aus N-hydroxyalkylsubstituierten Aspartamideinheiten sowie deren Acylierungsprodukten mit langkettigen Carbonsäurederivaten und gegebenenfalls aus restlichen Polysuccinimideinheiten bestehen. Derartige Verbindungen sind durch Hydrolyse, beispielsweise alkalische Hydrolyse mit Alkalimetallhydroxiden, der in der EP 0 458 079 A beschriebenen copolymeren Polysuccinimidderivate zugänglich.

Weiterhin können copolymere Asparaginsäurederivate eingesetzt werden, welche aus Asparaginsäureeinheiten mit freien Carbonsäure- bzw. Carboxylatseitenketten, gegebenenfalls aus Polysuccinimideinheiten bestehen sowie am Kettenende eingebaute einwertige Alkylalkohole und/oder Alkylamine enthalten. Derartige Verbindungen sind beispielsweise nach EP 0 650 995 A aus Maleinsäure oder Maleinsäureanhydrid, Ammoniak oder Ammoniumderivaten sowie den Alkylaminen und/oder -alkoholen zugänglich.

Die eingesetzten Polymeren können nachbehandelt werden, beispielsweise durch Behandlung mit Aktivkohle, Bleichung mit Oxidationsmitteln wie H₂O₂, Cl₂, Ozon, Natriumchlorit, Natriumhypochlorit etc oder Reduktionsmitteln wie z. B. NaBH₄ oder H₂ in Gegenwart von Katalysatoren.

Der Anteil der Polyasparaginsäurederivate in den erfindungsgemäßen kosmetischen Mitteln kann zwischen 0.1 und 40 Gew.-%, vorzugsweise 1-15 Gew.-% betragen, wobei der Gesamtanteil aller mit diesen in Kombination eingesetzten Tenside 1-95 Gew.-%, vorzugsweise 1-50 Gew.-% beträgt. Es sind feste, flüssige oder pastöse Zübereitungen möglich, beispielsweise Seifenstücke, Waschlotionen, Duschgele, Shampoos.

Die biologisch abbaubaren Polyasparaginsäurederivate mit einem naturnahen Polyaminosäurerückgrat sind ausgesprochen milde Tenside, welche in Kombination mit anionischen, kationischen, nichtionischen, zwitterionischen oder amphoteren Tensiden hervorragendes Schaumverhalten zeigen. Sie sind schäumend und stabilisieren andere tensidische Schäume. Mit Hilfe der hier beschriebenen Polyasparaginsäurederivate ist ein Zugang zu milden, leicht konservierbaren Tensidformulierungen möglich, ohne Einbußen oder Nachteile bezüglich Reinigungswirkung und Schaumverhalten in Kauf nehmen zu müssen. Durch die Auswahl der geeigneten Polyasparaginsäurederivate können die gewünschten Eigenschaften der Tensidformulierung (beispielsweise Schaum schwach, reich oder cremig, Viskosität, Betonung der pflegenden oder reinigenden Wirkung) erhalten bleiben oder sogar gefördert werden.

Die erfindungsgemäß in Kombination mit Polyasparaginsäurederivaten eingesetzten Tenside können beispielsweise anionische Tenside aus der Gruppe der Sulfate, Sulfonate, Carboxylate sowie Mischungen derselben sein. Die anionischen Gruppen können in neutralisierter Form vorliegen, mit kationische Gegenionen aus der Gruppe der Alkalimetalle, beispielsweise Natrium oder Kalium, Erdalkalimetalle (beispielsweise Magnesium oder Calcium), Ammonium oder sübstituiertem Ammonium, beispielsweise Tetraalkylammonium oder Mono/Di/Triethanolammonium. Eingesetzt werden beispielsweise Alkylbenzolsulfonate, α-Olefinsulfonate, α-sulfonierte Fettsäureester, Fettsäureglycerinestersulfate, Paraffinsulfonate, Alkylsulfate, Alkylpolyethersulfate, Sulfobernsteinsäurealkylester, Fettsäuresalze (Seifen), Fettsäureester der Polymilchsäure, N-Acylaminosäureester, N-Acyltaurate, Acylisethionate, Ethercarboxylate, Monoalkylphosphate, N-Acylaminosäurederivate wie N-Acylaspartate oder N-Acylglutamate, N-Acylsarcosinate und andere. Der Anteil an anionischen Tensiden in den erfindungsgemäßen Tensidzübereitungen kann zwischen 0 und 95 Gew.-% betragen.

Die erfindungsgemäß in Kombination mit Polyasparaginsäurederivaten eingesetzten Tenside können beispielsweise kationische Tenside, beispielsweise aus der Gruppe der quartären Ammoniumverbindungen, quaternisierten Proteinhydrolysate, Alkylamidoamine, quartären Esterderivate, quartären Silikone, quartären Zucker- und Polysaccharidderivate, und Mischungen daraus, sein.

Die erfindungsgemäß in Kombination mit Polyasparaginsäurederivaten eingesetzten Tenside können beispielsweise amphotere oder zwitterionische Tenside sein, welche sowohl anionische Gruppen (wie beispielsweise Carboxylat oder Sulfonat sowie Mischungen daraus) als auch entweder Amingruppen (bei amphoteren Tensiden) oder (bei zwitterionischen Tensiden) kationische Gruppen wie Ammonium, Phosphonium oder Sulfonium sowie Mischungen daraus tragen. Zu dieser Gruppe gehören beispielsweise Alkylbetaine, Alkylamidoalkylbetaine des Typs Cocoamidopropylbetain, Sulfobetaine, Phosphobetaine, Sultaine und Amidosultaine, Imidazoliniumderivate, Amphoglycinate, polyfunktionelle amphotere Tenside (Lomax-Typen). Der Anteil an amphoteren und/oder zwitterionischen Tensiden in den erfindungsgemäßen Tensidzübereitungen kann zwischen 0 und 95 Gew.-% betragen.

Die erfindungsgemäß in Kombination mit Polyasparaginsäurederivaten eingesetzten Tenside können beispielsweise nichtionische Tenside sein, ausgenommen Alkylpolyglucoside, Alkenylpolyglucoside und Fettsäure-N-alkylpolyhydroxyalkylamide. Die nichtionischen Tenside können beispielsweise aus der Verknüpfung von Alkylenoxidgruppen hydrophiler Natur, vorzugsweise auf der Basis von Ethylenoxid oder Ethylenoxid/Propylenoxid, mit hydrophoben Alkyl oder Arylresten hervorgehen. Beispiele sind oxethylierte Fettalkohole, oxethylierte Alkylphenole, oxethylierte Fettsäureester, oxethylierte Mono-, Di- oder Triglyceride oder Polyalkylenglykolfettsäureester. Andere nichtionische Tenside können aus der Gruppe der Zuckerester, beispielsweise Fettsäureester der Saccharose, Fructose oder des Methylglucosids, Sorbitolfettsäureester und Sorbitanfettsäureester (gegebenenfalls oxethyliert), Polyglycerinester, Fettsäurealkanolamide, langkettige tertiäre Aminoxide oder Phosphinoxide sowie Dialkylsulfoxide stammen. Hierbei ist insbesondere der Ausschluß der oben genannten zuckerbasierenden Tenside zu beachten. Die angegebenen Tensidtypen können alleine oder in Mischungen eingesetzt werden. Der Gehalt an nichtionischen Tensiden in den erfindungsgemäßen Tensidzubereitungen kann zwischen 0% und 75 Gew.-% betragen.

In vorteilhafter Weise umfassen die Tensidzubereitungen neben wenigstens einem Polyasparaginsäurederivat wenigstens ein weiteres Tensid aus der Gruppe der nichtionischen, kationischen, zwitterionischen oder amphoteren Tenside, oder zwei anionische Tenside oder wenigstens ein anionisches Tensid in Kombination mit wenigstens einem weiteren Tensid aus der Gruppe der anionischen, nichtionischen, kationischen, zwitterionischen oder amphoteren Tenside, oder wenigstens ein nichtionisches Tensid, wenigstens ein Polyasparaginsäurederivat sowie wenigstens ein weiteres Tensid aus der Gruppe der anionischen, nichtionischen, kationischen, zwitterionischen oder amphoteren Tenside. Auch sind Tensidzubereitungen, welche wenigstens ein amphoteres oder zwitterionisches Tensid, wenigstens ein Polyasparaginsäurederivat sowie wenigstens ein weiteres Tensid aus der Gruppe der anionischen, nichtionischen, zwitterionischen und amphoteren Tenside enthalten, in vorteilhafter Weise einsetzbar. In diesen Fällen beträgt die Konzentration der Polyasparaginsäurederivate bevorzugt 1 bis 40 Gew.-%, insbesondere 1 bis 15 Gew.-%, die der weiteren Tensidkomponenten jeweils 1 bis 80 Gew.-%, insbesondere 1 bis 50 Gew.-%.

Die erfindungsgemäßen Tensidzubereitungen können weitere Hilfs- und Zusatzstoffe enthalten wie beispielsweise: Wasser, Lösungsmittel z.B. aus der Gruppe der Alkohole, vorzugsweise der aliphatischen Alkohole, und Polyole wie beispielsweise Ethanol, Propanol, i-Propanol, Propylenglykol, Glycerin oder Polyethylenglykole, sowie Gemische daraus; Verdickungsmittel, beispielsweise Kaolin, Bentonit, Fettsäuren, höhere Fettalkohole, Stärke, Polyacrylsäure und deren Derivate, Cellulosederivate, Alginate; Trübungsmittel, beispielsweise Glykolesterderivate; Moisturizer, beispielsweise langkettige Fettsäuren oder deren Ester, flüssige, wasserlösliche Polyole wie Glycerin, Sorbitol, Polyethylenglokol, Propylenglykol, Milchsäure, biogene Aminosäuren, etc.; Emollients wie tierische und pflanzliche Öle, Carbonsäureester, Lanolin, Bienenwachs, Silicone; Polymere Agenzien zur Verbesserung des Hautgefühls wie beispielsweise nonionic guar gums, nichtionische Cellulosederivate wie Hydroxyethylcellulose und Carboxymethylcellulose; konditionierende, pflegende oder pharmazeutisch wirksame Bestandteile wie beispielsweise kationische oder amphotere Polymere, Proteine und Proteinderivate, Lanolinderivate, Panthothensäure, Betain, Polydimethylsiloxane oder deren Derivate, Sonnenschutzwirkstoffe wie beispielsweise p-Aminobenzoesäure und deren Derivate, Stilbenderivate etc.; sowie Lösungsvermittler, Stabilisatoren, Puffersubstanzen, Geruchsstoffe, und/oder Farbstoffe. Gegebenenfalls können auch zusätzlich dem Stand der Technik entsprechende Konservierungsmittel wie beispielsweise Parabene, Benzoesäure und -derivate, Methyldibromoglutaronitril, Diazolidinylharnstoff, Sorbinsäure, Phenoxyethanol und andere enthalten sein.

Die Polyasparaginsäurederivate enthaltenden Tensidzubereitungen lassen sich vorteilhaft anwenden in beispielsweise Haarshampoos, Duschbäder, Schaumbadznbereitungen, Hand-, Gesichts- und Intimreinigungslotionen, Flüssigseifen, Seifenstücken, Rasiercremes, Handwaschpasten, hautfreundlichen Geschirrspülmitteln, Reinigungsmitteln für glatte Oberflächen sowie in Zahncremes. Sie ergeben einen reichen, cremigen Schaum, sind leicht auswaschbar und zeichnen sich durch ihre Milde, leichte Konservierbarkeit und ihr angenehmes Hautgefühl aus.

### Beispiele:

### Beispiel 1: Poly(asparaginsäure-co-decylaspartat)

Poly(asparaginsäure-co-decylaspartat) wurde nach DE 195 45 678 A durch Öffnung von Polysuccinimid mit Methanol, nachfolgende Umesterung mit 0,3 Eguivalenten n-Decanol sowie Hydrolyse mit NaOH hergestellt. Veresterungsgrad (quant. C-NMR): 26% COOC₁₀H₂₁

### Beispiel 2

Verschiedene Tensidformulierungen gemäß der in der Tabelle angegebenen Zusammensetzung wurden hergestellt und die Überlebensrate unterschiedlicher Mikroorganismen in diesen Formulierungen bestimmt.

| Formulierung | A Gew.% | B Gew.% | C Gew.% |
|---|---|---|---|
| Texapon® (70% Natriumlaurylethersulfat, Henkel) | 9,5 | 9,5 | 9,5 |
| Tego®Betain F50 (Cocoamidopropylbetain, Th. Goldschmidt) | 18,8 | 18,8 | 18,8 |
| Testtensid I oder II | 13,0 | 13,0 | 13,0 |
| NaCl (25%) | 6,8 | 6,8 | 6,8 |
| Tegosoft® GC (PEG-7 Glycerylcocoat, Th. Goldschmidt) | 4,5 | 3,6 | 2,8 |
| Antil® 171 (PEG-18 Glyceryloleat/cocoat, Th. Goldschmidt) | 2,4 | 2,4 | 2,4 |
| Sorbitol (70%) | 30,7 | 24,9 | 18,9 |
| Wasser | 14,3 | 21,0 | 28,6 |
| pH | 5,0 | 5,0 | 5,0 |
| Testtensid I: Plantaren® 1200 (Laurylpolyglucose, 51,5%ig, Henkel) (APG) Testtensid II: Poly(asparaginsäure-co-decylaspartat), 51,5%ig in Wasser, pH 5,0 (Polyaspartat) | | | |

### Überlebensrate von C. albicans (Keimzahl)

| Formulierung | 0. Tag | 1. Tag | 7. Tag |
|---|---|---|---|
| A I (APG) | 1,1 x10⁵ | 200 | <1 |
| A II (Polyaspartat) | 1,1 x10⁵ | <1 | <1 |
| B I (APG) | 1,1 x10⁵ | 410 | <1 |
| B II (Polyaspartat) | 1,1 x10⁵ | <1 | <1 |
| C I (APG) | 1,1 x10⁵ | 4400 | 90 |
| C II (Polyaspartat) | 1,1 x10⁵ | <1 | <1 |

### Überlebensrate von A. niger (Keimzahl)

| Formulierung | 0. Tag | 1. Tag | 7. Tag |
|---|---|---|---|
| A I (APG) | 3,8 x10⁴ | 7,0 x10⁵ | 6,0 x10⁴ |
| A II (Polyaspartat) | 3,8 x10⁴ | <1 | <1 |
| B I (APG) | 3,8 x10⁴ | 4,5 x10⁵ | 9,0 x10⁴ |
| B II (Polyaspartat) | 3,8 x10⁴ | <1 | <1 |
| C I (APG) | 3,8 x10⁴ | 6,0 x10⁴ | 1,6 x10⁵ |
| C II (Polyaspartat) | 3,8 x10⁴ | <1 | <1 |

### Überlebensrate von Mischkeimen aus S. aureus und Ps. aeruginosa (Keimzahl)

| Formulierung | 0. Tag | 1. Tag | 7. Tag |
|---|---|---|---|
| A I (APG) | 5,1 x10⁶ | 2,9 x10³ | <1 |
| A II (Polyaspartat) | 5,1 x10⁶ | 20 | <1 |
| B I (APG) | 5,1 x10⁶ | 3,3 x10³ | <1 |
| B II (Polyaspartat) | 5,1 x10⁶ | <1 | <1 |
| C I (APG) | 5,1 x10⁶ | 4,3 x10⁴ | 560 |
| C II (Polyaspartat) | 5,1 x10⁶ | <1 | <1 |

Diese Ergebnisse belegen die keimwachstumshemmende Wirkung der Polyasparaginsäuretenside und die gute Konservierbarkeit der Formulierungen im Vergleich zu APG-haltigen Formulierungen.

## Patentansprüche

1. Tensidische Zubereitungen für Reinigungsmittel und/oder kosmetische Mittel, enthaltend ein oder mehrere hydrophob modifizierte Polyasparaginsäurederivate und ein oder mehrere weitere Tenside aus der Gruppe der nichtionischen, kationischen, amphoteren oder zwitterionischen Tenside sowie deren Mischungen oder wenigstens ein anionisches Tensid, in Kombination mit wenigstens einem anionischen, kationischen, nichtionischen, zwitterionischen und/oder amphoteren Tensid , ausgenommen zuckerbasierende Tenside aus den Klassen der Alkyl- oder Alkenylpolyglucoside oder Fettsäure-N-alkyl-polyhydroxyalkylamide, neben üblichen Hilfs- und/oder Zusatzstoffen.

2. Tensidische Zubereitungen für Reinigungsmittel und/oder kosmetische Mittel, enthaltend ein oder mehrere hydrophob modifizierte Polyasparaginsäurederivate und wenigstens zwei weitere Tenside aus der Gruppe der anionischen Tenside und gegebenenfalls ein oder mehrere weitere Tenside aus der Gruppe der anionischen, kationischen, nichtionischen amphoteren oder zwitterionischen Tenside, sowie deren Mischungen daraus, ausgenommen zuckerbasierende Tenside aus den Klassen der Alkyl- oder Alkenylpolyglucoside oder Fettsäure-N-alkyl-polyhydroxyalkylamide,neben üblichen Hilfs- und/oder Zusatzstoffen.

3. Tensidzubereitungen nach Anspruch 1 oder 2, wobei die Polyasparaginsäurederivate aus einem überwiegend aus Asparaginsäureeinheiten bestehenden Polyaminosäurerückgrat bestehen, welches partiell durch hydrophobe Alkylgruppen mit 6-24 C-Atomen modifiziert ist.

4. Tensidzubereitungen nach einem der Ansprüche 1 bis 3, enthaltend copolymere Polyasparaginsäurederivate zu wenigstens 75 Gew.-% aus Asparaginsäureeinheiten und/oder deren Metall- oder Ammoniumsalzen sowie aus Asparaginsäuregruppen, welche mit einem oder mehreren gleichen oder verschiedenen Alkoholen mit 1-24 C-Atomen verestert sind, wobei wenigstens eine Estergruppe eines Alkohols mit 6-18 C-Atomen enthalten ist.

5. Tensidzubereitungen nach einem oder mehreren der Ansprüche 1 bis 4, enthaltend copolymere Polyasparaginsäurederivate, bestehend aus einem überwiegend aus Asparaginsäureeinneiten bestehenden Polyaminosäurerückgrat, welche mit einem oder mehreren gleichen oder verschiedenen Alkoholen mit 1 bis 5 C-Atomen verestert sind.

6. Tensidzübereitungen nach einem der Ansprüche 1 bis 5, enthaltend copolymere Polyasparaginsäureester, welche durch Ringöffnung von Polysuccinimid mit Alkoholen mit 1 bis 24 C-Atomen, sowie gebenenfalls durch nachträgliche Umesterung der Produkte mit Alkoholen mit 2 bis 30 C-Atomen, und gegebenenfalls abschliessende Hydrolyse zugänglich sind.

7. Tensidzubereitungen nach einem der Ansprüche 1 bis 6, enthaltend copolymere Polyasparaginsäureester, welche durch Veresterung von Polyasparaginsäure oder asparagin-säurereichen Polyaminosäuren und deren Carboxylanaloga mit Alkoholen mit 1 bis 24 C-Atomen, sowie gegebenenfalls durch nachträgliche Umesterung der Produkte mit Alkoholen mit 6 bis 24 C-Atomen, und gegebenenfalls abschliessende Hydrolyse zugänglich sind.

8. Tensidzubereitungen nach Anspruch 1 oder 2, enthaltend copolymere Polyasparaginsäurederivate aus Polyasparaginsäureeinheiten, deren Salzen oder Polysuccinimideinheiten, die endständig an der Polymerkette mit einwertigen Alkoholen oder Aminen mit 6 bis 24 C-Atomen verknüpft sind.

9. Tensidzubereitungen nach einem der Ansprüche 1 bis 3, enthaltend oberflächenaktive copolymere Polyasparaginsäurederivate aus Asparaginsäureeihheiten mit freien Carboxyl- und Carboxylatresten, aus N-hydroxyalkylsübstituierten Aspartamideinheiten sowie deren Acylierungsprodukten mit Fettsäurederivaten und gegebenenfalls aus weiteren Polysuccinimideinheiten.

10. Verwendung der Tensidzübereitungen nach einem oder mehreren der Ansprüche 1 bis 9 in milden, schäumenden Tensidsystemen für Shampoos, Waschlotionen und Reinigungsmittel für Gesicht, Haar, Haut und Intimbereich, Rasiercremes und - lotionen, Flüssigseifen, Geschirrspülmitteln, Reinigungsmitteln für glatte Oberflächen, Seifenstücken, Schaumbädern, Duschgelen sowie in Zahncremes und/oder Mundspülungen.
